# EUROPEAN PATENT SPECIFICATION

(11) **EP 2 994 198 B1**
(45) Date of publication and mention of the grant of the patent: **05.06.2019**
(21) Application number: 14718395.8
(22) Date of filing: 16.04.2014
(51) Int. Cl.: A61Q 5/02, A61Q 5/12, A61Q 5/00, A61K 8/46, A61K 8/36, A61Q 19/08, A61Q 19/10

(54) **HAIR TREATMENT COMPOSITION**
ZUSAMMENSETZUNG ZUR HAARBEHANDLUNG
COMPOSITIONS DE TRAITEMENT CAPILLAIRE

(30) Priority: 09.05.2013 WO PCT/CN2013/075418; 26.06.2013 EP 13173685
(43) Date of publication of application: 16.03.2016
(73) Proprietor: Unilever N.V., 3013 AL Rotterdam (NL); Unilever PLC, London, Greater London EC4Y 0DY (GB)
(72) Inventor: CAO, Qunhua, Changing District Shanghai 200335 (CN); JAYASWAL, Amit, Changing District Shanghai 200335 (CN); LING, Zihui, Changing District Shanghai 200335 (CN)
(74) Representative: Fijnvandraat, Arnoldus
(86) International application number: PCT/EP2014/057761
(87) International publication number: WO 2014/180640

(56) References cited:
- WO-A1-2006/002892
- WO-A1-2009/030594
- WO-A2-2009/077495
- WO-A2-2010/034721
- WO-A2-2013/150300
- US-A- 5 225 097
- US-A- 5 227 086
- US-A1- 2008 153 727
- US-B1- 6 183 757

## Description

### FIELD OF THE INVENTION

The present invention relates to a hair treatment composition. In particular, the present invention relates to a hair treatment composition comprising aliphatic acid comprising C₈₋₂₄ hydroxyl substituted aliphatic acid, wherein the aliphatic acid comprises 12-hydroxystearic acid; a fatty acyl isethionate product which product comprises 40 to 80% of one or more fatty acyl isethionate salts by weight of the product and 15 to 50% of one or more free fatty acids and/or the salts of the free fatty acid by weight of the product; and at least 30% of water by weight of the composition, wherein the fatty acid is substantially different from the aliphatic acid.

### BACKGROUND OF THE INVENTION

Hair treatment compositions are well known and have been widely used for long time. Beside the basic function of cleaning, the hair treatment composition can also deliver the benefit of conditioning, anti-dandruff, cooling etc. by including the corresponding functional additive into the composition.

However, when some beneficial agents are included into the composition, the interaction between the ingredients may become very complicated. Thus, there are some difficulties to formulate a stable composition, especially for example when aliphatic acid comprising C₈₋₂₄ hydroxyl substituted aliphatic acid, wherein the aliphatic acid comprises 12-hydroxystearic acid is incorporated. The addition of such beneficial agents may induce instability of the hair treatment product.

US patent application US 2008/0153727 A1 (Unilever) discloses novel liquid cleansing compositions which are formed by combination of (a) surfactant liquid crystal inducers, which helps formation of liquid crystals rather than solid crystals; and (b) liquid crystal modifier.

International patent application WO 2006/002892 A1 (Unilever) discloses a mild syndet toilet bar with excellent physical and processing properties for cleansing the human body, such as the skin and hair.

US patent US 6,183,757, B1 (Procter & Gamble Company) is said to disclose a mild, rinse-off personal cleaning compositions which provide enhanced antimicrobial effectiveness.

International patent application WO 2009/030594 A1 (Unilever) discloses a personal care skin or hair cleansing compositions comprising fatty acyl isethionate surfactants, as the primary surfactant, at a level of at least 60% of total fatty isethionate surfactant product and other synthetic co-surfactants in the personal skin or hair liquid cleansing compositions.

International patent application WO 2009/077495 A2 (Unilever) discloses novel liquid cleansing compositions containing fatty acryl isethionate surfactant product which compositions are stabilised with a specific combination of long chain and short chain fatty acids/fatty soaps to increase the viscosity of the liquid composition at elevated temperatures.

International patent application WO 2010/034721 A2 (Unilever) discloses a personal care skin or hair cleansing compositions comprising fatty acyl isethionate surfactant product as the primary component at a level of at least 50 wt. % of total fatty isethionate surfactant product and other synthetic co-surfactants in the personal skin or hair liquid cleansing compositions.

US patent US 5,225,097 (Procter & Gamble Company) is said to disclose a firm, low smear, ultra mild, weakly acidic skin pH personal cleansing freezer bar comprising by weight of said bar: a sum total of from about 10% to about 50% of essentially free carboxylic acid, preferably myristic acid, behenic acid, or 12-hydroxy stearic acid; from about 15% to about 65% of a water-soluble organic anionic and/or nonionic bar firmness aid, preferably sodium cocoyl isethionate or sodium lauroyl isethionate; and from about 15% to about 40% water.

US patent US 5,227,086 (Procter & Gamble Company) is said to disclose a firm, ultra mild, weakly acidic skin pH cleansing bar comprising by weight of said bar: from about 5% to about 50% of essentially free monocarboxylic acid; from about 15% to about 65% of a water-soluble organic anionic and/or non-ionic bar firmness aid; and from about 15% to about 55% water.

We have recognized a need to develop a stable hair treatment composition with incorporation of aliphatic acid comprising C₈₋₂₄ hydroxyl substituted aliphatic acid, wherein the aliphatic acid comprises 12-hydroxystearic acid. It was surprisingly found that the hair treatment composition is quite stable when incorporating aliphatic acid comprising C₈₋₂₄ hydroxyl substituted aliphatic acid, wherein the aliphatic acid comprises 12-hydroxystearic acid; and a fatty acyl isethionate product together into the composition.

### SUMMARY OF THE INVENTION

In a first aspect, the present invention is directed to a hair treatment composition comprising aliphatic acid comprising C₈₋₂₄ hydroxyl substituted aliphatic acid, wherein the aliphatic acid comprises 12-hydroxystearic acid; a fatty acyl isethionate product which product comprises 40 to 80% of one or more fatty acyl isethionate salts by weight of the product and 15 to 50% of one or more free fatty acids and/or the salts of the free fatty acid by weight of the product; and at least 30% of water by weight of the composition, wherein the fatty acid is substantially different from the aliphatic acid.

In a second aspect, the present invention is directed to a process for preparing a hair treatment composition of the present invention comprising the steps of premixing aliphatic acid comprising C₈₋₂₄ hydroxyl substituted aliphatic acid, wherein the aliphatic acid comprises 12-hydroxystearic acid, with a fatty acyl isethionate product which product comprises 40 to 80% of one or more fatty acyl isethionate salts by weight of the product and 15 to 50% of one or more free fatty acids and/or the salts of the free fatty acid by weight of the product; and combining the premix with the remaining component of the hair treatment composition, wherein the fatty acid is substantially different from the aliphatic acid.

All other aspects of the present invention will more readily become apparent upon considering the detailed description and examples which follow.

### DETAILED DESCRIPTION

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The aliphatic acid comprises 12-hydroxystearic acid.

The aliphatic acid is free aliphatic acid. Such compounds are commercially available from suppliers like Vertellus Specialties Inc. Welch, Home & Clark Co. Inc. as well as Croda Chemicals.

The amount of aliphatic acid in the composition is preferably from 0.0001 to 7% by weight of the composition, more preferably from 0.001 to 5%, even more preferably from 0.005 to 3%, still even more preferably from 0.01 to 1% and most preferably from 0.05 to 0.6% by weight of the composition.

The fatty acyl isethionate product suitable for use in the present invention typically may be prepared by the reaction of at least one fatty acid of formula R²C(O)OH with at least one hydroxyalkylsulfonic acid salt of the formula HOR³SO₃M, wherein R² which is selected from monovalent, linear or branched, saturated or unsaturated hydrocarbon radicals, R³ is selected from divalent, linear or branched, saturated hydrocarbon radicals having 2 to 6 carbon atoms, and M is a solubilising cation (such as lithium, sodium, potassium, ammonium or substituted ammonium).

Suitable fatty acids for use in the preparation of the fatty acyl isethionate product include those in which R² is a linear or branched, saturated or unsaturated hydrocarbon radical having 5 to 21 carbon atoms. Such fatty acids may be naturally occurring or synthetic or a mixture thereof. Naturally occurring fatty acids are usually mixtures of acids having a hydrocarbon chain of varying length. For example, "coconut fatty acid" as used herein is a mixture containing a range of carboxylic acids in which the R² carbon chain length ranges from 7 to 17 carbon atoms, with some unsaturation which may be removed by hydrogenation. Thus, hydrogenated coconut fatty acid is a mixture containing a range of carboxylic acids in which the R⁴ carbon chain length ranges from 7 to 17 carbon atoms, mostly lauric and myristic acids, together with some capric and caprylic acids, and contains very little, if any, unsaturation.

Preferred fatty acids are those in which R² is a linear or branched, saturated or unsaturated hydrocarbon radical having 7 to 17 carbon atoms.

Specific examples of preferred fatty acids include coconut fatty acid (R² having 7 to 17 carbon atoms), hydrogenated coconut fatty acid (R² having 7 to 17 carbon atoms), lauric acid (R² having 11 carbon atoms), palmitic acid (R² having 15 carbon atoms), palm kernel fatty acid (R² having 7 to 17 carbon atoms), oleic acid (unsaturated R² having 17 carbon atoms), stearic acid (saturated R² having 17 carbon atoms), tallow fatty acid (partially unsaturated R²mainly having 15 and 17 carbon atoms), and hydrogenated tallow fatty acid.

Mixtures of any of the above described fatty acids may also be used.

Suitable hydroxyalkylsulfonic acid salts for use in the preparation of the fatty acyl isethionate product include those in which R³ has the general formula of -CH(R⁵)-CH(R⁶)-, in which R⁵ and R⁶ are each independently selected from -H, -CH₃ and -CH₂CH₃.

Specific examples of preferred hydroxyalkylsulfonic acid salts include hydroxyethylsulfonic acid salts such as sodium hydroxyethylsulfonate.

Other specific examples of preferred hydroxyalkylsulfonic acid salts include alkyl-substituted hydroxyethylsulfonic acid salts such as the sodium salts of methyl and/or ethyl-substituted hydroxyethylsulfonic acid salts, for example sodium 1-methyl 2-hydroxy ethane 1-sulfonate, sodium 2-methyl, 2-hydroxy ethane 1-sulfonate, sodium 1-ethyl 2-hydroxy ethane 1-sulfonate and sodium 2-ethyl, 2-hydroxy ethane 1-sulfonate.

Mixtures of any of the above described hydroxyalkylsulfonic acid salts may also be used.

In a typical process used to prepare the fatty acyl isethionate product (termed the "direct esterification route"), the fatty acid and hydroxyalkylsulfonic acid salt are mixed and heated in the presence of a metal catalyst. Generally, an excess of fatty acid is used in order to shift the reaction equilibrium to fatty acyl isethionate synthesis. Thus, the molar ratio of fatty acid to hydroxyalkylsulfonic acid salt may range from 1.3:1 to 1.1:1.

The resulting fatty acyl isethionate product is usually termed "directly esterified fatty isethionate" (or DEFI) and may be characterized as a mixture of 40 to 80% fatty acyl isethionate by weight of the product(formed from the reaction), 15 to 50% free fatty acids and/or the salts of the free fatty acid by weight of the product, in addition to unreacted hydroxyalkylsulfonic acid salt (generally at levels less than 5% by weight of the product) and traces of other impurities (generally at levels less than 2% by weight of the product).

Typically, the fatty acyl isethionate product the fatty acyl isethionate salts have the formula of R²C(O)OR³SO₃M and the fatty acids/salts have the formula of R⁴C(O)OX, wherein R² and R⁴ are independently selected from monovalent, linear or branched, saturated or unsaturated hydrocarbon radicals, preferably having 5 to 31 carbon atoms, more preferably having 5 to 21 carbon atoms and even more preferably having 7 to 17 carbon atoms, R³ is independently selected from divalent, linear or branched, saturated hydrocarbon radicals preferably having 2 to 6 carbon atoms, M is a solubilising cation, preferably M is sodium, and X is hydrogen or a solubilising cation, preferably X is H or sodium. Preferably at least one R² is same as R⁴.

A preferred class of fatty acyl isethionate product for use in the present invention comprises:
(i) from 65 to 80 wt% (by weight based on the total weight of the fatty acyl isethionate product) of one or more fatty acyl isethionate salts of general formula R²C(O)OR³SO₃M, in which R² is a linear or branched, saturated or unsaturated hydrocarbon radical having 7 to 17 carbon atoms, R² is -CH₂CH₂- or -CH(CH₃)-CH₂- , and M is a solubilising cation such as sodium; and
(ii) from 15 to 30wt% (by weight based on the total weight of the fatty acyl isethionate product) of one or more fatty acids of formula R²C(O)OX, wherein R¹ is a linear or branched, saturated or unsaturated hydrocarbon radical having 7 to 17 carbon atoms and X is hydrogen or a solubilising cation such as sodium.

Most preferably the one or more fatty acyl isethionate salts (i) are selected from sodium cocoyl isethionate, sodium cocoyl methyl isethionate, sodium cocoyl ethyl isethionate and mixtures thereof.

Most preferably the one or more fatty acids (ii) may be defined as a mixture (for example of coconut fatty acid and stearic acid and/or their corresponding sodium salts), in which the content of long chain material (typically C16 to C18) is at least 25 wt% by weight based on the total weight of the fatty acids (b).

Examples of commercial fatty acyl isethionate products that are particularly useful in the invention are DEFI flakes and Dove® cleansing bar noodles produced by Unilever. DEFI flakes typically contain about 68 to 80% of sodium fatty acyl isethionate by weight of the product and 15 to 30% of free fatty acid by weight of the product. More than 25% and no more than 35% of fatty acyl group of the resulting fatty acyl isethionate by weight based on the total fatty acyl isethionate have 16 to 18 carbon atoms; and more than 60% of the free fatty acid by weight based on the total free fatty acids have 16 to 18 carbon atoms. Dove® cleansing bar noodles are mixtures of DEFI flakes described above and long chain (mainly C₁₆ and C₁₈) fatty acid and fatty soap which contain about 40 to 55% of fatty acyl isethionate by weight of the product and 30 to 40% of fatty acid and fatty soap by weight of the total product.

The total amount of fatty acyl isethionate product in compositions of the invention preferably ranges from 0.0001 to 20 wt%, more preferably from 0.001 to 8 wt%, even more preferably from 0.01 to 3% and most preferably from 0.05 to 0.5% (based on the total weight of the composition).

In a preferred embodiment the composition further comprises a cleansing surfactant.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R₂OSO₃M and R₁O (C₂H₄OₓSO₃M, wherein R₂ is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R₂ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 wt% to 25 wt% of the total composition, more preferably from 3 wt% to 18 wt%, most preferably from 6 wt% to 15 wt% of the total composition.

The total amount of anionic cleansing surfactant in compositions of the invention generally ranges from 0.5 wt% to 45 wt%, more preferably from 1.5 wt% to 20 wt%.

Compositions of the invention may contain non-ionic surfactant. Most preferably non-ionic surfactants are present in the range 0 to 5 wt%.

Nonionic surfactants that can be included in compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)ₙOH, where R is an alkyl chain of C12 to C15, and n is 5 to 9.

Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92/06154 and US 5,194,639, and the N-alkoxy polyhydroxy fatty acid amides, such as C₁₀-C₁₈ N-(3-methoxypropyl) glucamide.

Amphoteric or zwitterionic surfactant can be included in an amount ranging from 0.5 wt% to about 8 wt%, preferably from 1 wt% to 4 wt% of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The composition may comprise other actives for hair treatment product, for example, conditioning agent, cationic deposition polymer, anti-dandruff agent, suspending agent, or a mixture thereof.

Hair treatment compositions according to the invention such as shampoos and conditioners suitably contain conditioning agents such as silicone conditioning agents and non-silicone oily conditioning agents.

Suitable silicone conditioning agents include polydiorganosiloxanes, in particular polydimethylsiloxanes which have the CTFA designation dimethicone. Also suitable for use in compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning. Also suitable are functionalised silicones, particularly amino-functionalised silicones.

Suitable non-silicone oily conditioning agents are selected from hydrocarbon oils, fatty esters and mixtures thereof.

The further conditioning agent is suitably present in shampoo or conditioner compositions at a level of from 0.05 to 10, preferably from 0.2 to 5, more preferably from about 0.5 to 3 percent by total weight of further conditioning agent based on total weight of the composition.
it is preferred that the composition comprises a cationic deposition polymer, which may assist in deposition of cooling active and/or other active ingredients in the composition. Preferably, the cationic deposition polymer is (or comprises) cationic polygalactomannan, especially guar or cassia derived polygalactomannan modified with hydroxypropyl trimonium chloride.

It is highly preferred that compositions according to the invention should contain from 0.01% to 2% wt. of the composition cationic deposition polymer, more preferably from 0.05 to 0.5% wt. and most preferably from 0.08 to 0.25% by weight of the composition.

Antidandruff agents are compounds that are active against dandruff and are typically antimicrobial agents and preferably antifungal agents.

Antifungal agents typically display a minimum inhibitory concentration of about 50 mg/ml or less against *Malassezia spp.*

Suitable antidandruff agents include compounds selected from azole based antifungal agents, octopirox, metal pyrithione salts, and mixtures thereof. The preferred azole based antifungal agents are ketoconazole and climbazole. Preferred metal pyrithione salts are zinc, copper, silver and zirconium pyrithione. The most preferred is zinc pyrithione.

Preferably, the antidandruff agent is present at from 0.01 to 5% wt. of the composition, more preferably from 0.1 to 2.5% wt. of the composition.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a composition of the invention at levels of from 0.1 to 10%, preferably from 0.5 to 6%, more preferably from 0.9 to 4% by total weight of suspending agent based on the total weight of the composition.

The composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

The composition comprises at least 30% of water by weight of the composition, more preferably from 35 to 95%, even more preferably from 45 to 88%, still even more preferably from 55 to 82%, most preferably from 65 to 80% by weight of the total composition.

Compositions of the invention are primarily intended for topical application to at least a portion of the hair of an individual, either in rinse-off or leave-on compositions, for the treatment of dry and/or wet, damaged and/or unmanageable hair. Preferably, the composition is a shampoo.

Preferably, the composition has a viscosity of less than 200,000 centipoise. More preferably, the composition has a viscosity of no greater than 50,000 centipoise, even more preferably no greater than 20,000 centipoise, and most preferably no greater than 10,000 centipoise. It is preferred that the composition has a viscosity of at least 10 centipose. More preferably, the composition has a viscosity of at least 200 centipose, even more preferably at least 1000 centipose and still even more preferably at least 3000 centipose. The viscosity of the present invention is taken at 30°C with a Brookfield Viscometer, Spindle No. 5 at a speed of 20 rpm.

The composition may be prepared by any method suitable for hair treatment composition. However, it is preferred that the process for preparing a hair treatment composition of the present invention comprising the steps of:
i. premixing aliphatic acid comprising C₈₋₂₄ hydroxyl substituted aliphatic acid, wherein the aliphatic acid comprises 12-hydroxystearic acid, with a fatty acyl isethionate product which product comprises 40 to 80% of one or more fatty acyl isethionate salts by weight of the product and 15 to 50% of one or more free fatty acids and/or the salts of the free fatty acid by weight of the product and;
ii. combining the premix with the remaining component(s) of the hair case composition;
wherein the fatty acid is substantially different from the aliphatic acid, aliphatic acid.

Preferably, water is premixed together with aliphatic acid and fatty acyl isethionate product in step (i) to make the premix well dispersed. It is also preferred that step (i) is carried out at temperature of at least 40 degrees, more preferably at 50 to 95 degrees. Generally, stirring is used for making the component in premix well mixed.

The invention will now be described with reference to the following non-limiting examples.

### EXAMPLE 1

**Table 1**

| **Ingredient % wt** | **1** | **A** |
|---|---|---|
| Aqua | Balance to 100% | Balance to 100% |
| Sodium Laureth Sulfate | 14.00 | 14.00 |
| Cocamidopropyl Betaine | 1.60 | 1.60 |
| Perfume | 0.70 | 0.70 |
| Dimethiconol | 0.9 | 0.9 |
| Dimethicone | 0.6 | 0.6 |
| Carbomer | 0.60 | 0.60 |
| Sodium Chloride | 0.40 | 0.40 |
| Sodium Hydroxide | 0.31 | 0.31 |
| Zinc Pyrithione | 0.25 | 0.25 |
| Guar Hydroxypropyltrimonium Chloride | 0.20 | 0.20 |
| Pearliser | 0.60 | 0.60 |
| Zinc Sulfate | 0.10 | 0.10 |
| Emotives | 0.34 | 0.34 |
| 12-hydroxystearic acid | 0.10 | 0.10 |
| Dove® cleansing bar noodles* | 0.10 | --- |
| Stearic acid | --- | 0.10 |
| Preservative | 0.31 | 0.31 |
| Colorant | 0.00034 | 0.00034 |

| | | |
|---|---|---|
| * The Dove® cleansing bar noodles is Sodium Lauroyl Isethionate, Sodium Palmitate, Stearic Acid, Lauric Acid, Sodium Isethionate and Water produced in-house by Unilever | | |

The compositions in Table 1 were prepared by following procedure. Dove® cleansing bar noodles (or stearic acid), 12-hydroxystearic acid, and 5% of water were premixed at temperature of 80 °C under stirring of 150 rpm until the pre-mixture was well mixed. Then, the pre-mixture was cooled down to room temperature. A normal procedure for shampoo composition was used to combine the pre-mixture with other ingredient in shampoo.

Both composition 1 and A were used to conduct stability test. The stability test was conducted for 3 months at temperature of 50 °C. Then, the compositions were checked whether there are colour changes, sniff changes, and detectable phase separation. It was surprisingly found that composition 1 was stable and passed the stability test and composition A failed in the stability test. It was manifested that the composition of the present invention comprising fatty acyl isethionate product was more stable than the composition comprising fatty acid merely.

## Claims

1. A hair/scalp treatment composition comprising:
a) aliphatic acid comprising C₈₋₂₄ hydroxyl substituted aliphatic acid, wherein the aliphatic acid comprises 12-hydroxystearic acid;
b) a fatty acyl isethionate product which product comprises 40 to 80% of one or more fatty acyl isethionate salts by weight of the product and 15 to 50% of one or more free fatty acids and/or the salts of the free fatty acid by weight of the product; and
c) at least 30% of water by weight of the composition;
wherein the fatty acid is substantially different from the aliphatic acid.

2. The composition according to claim 1 wherein the aliphatic acid is present in amount of from 0.0001 to 5% by weight of the composition, preferably from 0.01 to 1% by weight.

3. The composition according to any one of the preceding claims wherein the fatty acyl isethionate product is present in amount of 0.001 to 10% by weight of the composition.

4. The composition according to any one of the preceding claims wherein the fatty acyl isethionate salts have the formula of R²C(O)OR³SO₃M and the fatty acids/salts have the formula of R⁴C(O)OX, wherein R² and R⁴ are independently selected from monovalent, linear or branched, saturated or unsaturated hydrocarbon radicals having 5 to 31 carbon atoms, R³ is independently selected from divalent, linear or branched, saturated hydrocarbon radicals having 2 to 6 carbon atoms, M is a solubilising cation, and X is hydrogen or a solubilising cation.

5. The composition according to claim 4 wherein R² and R⁴ are independently selected from monovalent, linear, saturated or unsaturated hydrocarbon radicals having 5 to 24 carbon atoms, and M is sodium, and X is H or sodium.

6. The composition according to claim 4 or claim 5, wherein at least one R² is same as R⁴.

7. The composition according to any one of the preceding claim wherein the free fatty acid is saturated fatty acid without heteroatom substitution.

8. The composition according to any one of the preceding claims wherein the composition further comprises anionic surfactant, preferably sodium lauryl sulphate and/or sodium lauryl ether sulphate.

9. The composition according to any one of the preceding claims wherein the composition comprise at least one selected from hair conditioning agent, cationic deposition polymer, anti-dandruff active, suspending agent, and a combination thereof.

10. The composition according to any one of the preceding claims wherein the composition is a shampoo.

11. A process for preparing a hair/scalp treatment composition comprising the steps of:
i. premixing aliphatic acid wherein the aliphatic acid comprising C₈₋₂₄ hydroxyl substituted aliphatic acid, wherein the aliphatic acid comprises 12-hydroxystearic acid, with a fatty acyl isethionate product which product comprises 40 to 80% of one or more fatty acyl isethionate salts by weight of the product and 15 to 50% of one or more free fatty acids and/or the salts of the free fatty acid by weight of the product; and
ii. combining the premix the remaining component(s) of the hair treatment composition;
wherein the fatty acid is substantially different from the aliphatic acid, aliphatic acid.

## Patentansprüche

1. Zusammensetzung zur Behandlung von Haar/Kopfhaut, umfassend:
a) aliphatische Säure, umfassend C₈-C₂₄-Hydroxyl-substituierte aliphatische Säure, wobei die aliphatische Säure 12-Hydroxystearinsäure umfasst;
b) ein Fettacylisethionat-Produkt, welches Produkt 40 bis 80% eines oder mehrerer Fettacylisethionat-Salze, bezogen auf das Gewicht des Produkts, und 15 bis 50% eines oder mehrerer freier Fettsäuren und/oder der Salze der freien Fettsäure, bezogen auf das Gewicht des Produkts, umfasst; und
c) mindestens 30% Wasser, bezogen auf das Gewicht der Zusammensetzung;
wobei sich die Fettsäure im Wesentlichen von der aliphatischen Säure unterscheidet.

2. Zusammensetzung nach Anspruch 1, wobei die aliphatische Säure in einer Menge von 0,0001 bis 5%, bezogen auf das Gewicht der Zusammensetzung, vorzugsweise von 0,01 bis 1 Gewichts-%, vorliegt.

3. Zusammensetzung nach irgendeinem der vorhergehenden Ansprüche, wobei das Fettacylisethionat-Produkt in einer Menge von 0,001 bis 10%, bezogen auf das Gewicht der Zusammensetzung, vorliegt.

4. Zusammensetzung nach irgendeinem der vorhergehenden Ansprüche, wobei die Fettacylisethionat-Salze die Formel R²C(O)OR³SO₃M und die Fettsäuren/Salze die Formel R⁴C(O)OX aufweisen, wobei R² und R⁴ unabhängig voneinander unter einwertigen, linearen oder verzweigten, gesättigten oder ungesättigten Kohlenwasserstoff-Radikalen, die 5 bis 31 Kohlenstoffatome aufweisen, ausgewählt sind, R³ unabhängig aus zweiwertigen, linearen oder verzweigten, gesättigten Kohlenwasserstoff-Radikalen, die 2 bis 6 Kohlenstoffatome aufweisen, ausgewählt ist, M ein löslich machendes Kation ist und X Wasserstoff oder ein löslich machendes Kation ist.

5. Zusammensetzung nach Anspruch 4, wobei R² und R⁴ unabhängig voneinander unter einwertigen, linearen, gesättigten oder ungesättigten Kohlenwasserstoff-Radikalen, die 5 bis 24 Kohlenstoffatome aufweisen, ausgewählt sind und M Natrium ist und X H oder Natrium ist.

6. Zusammensetzung nach Anspruch 4 oder Anspruch 5, wobei mindestens ein R² das Gleiche wie R⁴ ist.

7. Zusammensetzung nach irgendeinem vorhergehenden Anspruch, wobei die freie Fettsäure eine gesättigte Fettsäure ohne Heteroatom-Substitution ist.

8. Zusammensetzung nach irgendeinem der vorhergehenden Ansprüche, wobei die Zusammensetzung ferner anionisches Tensid, vorzugsweise Natriumlaurylsulfat und/oder Natriumlaurylethersulfat, umfasst.

9. Zusammensetzung nach irgendeinem der vorhergehenden Ansprüche, wobei die Zusammensetzung mindestens eines umfasst, ausgewählt aus einem Haarkonditioniermittel, einem kationischen Abscheidepolymer, einem Antischuppenwirkstoff, einem Suspendiermittel und einer Kombination davon.

10. Zusammensetzung nach irgendeinem der vorhergehenden Ansprüche, wobei die Zusammensetzung ein Shampoo ist.

11. Verfahren zur Herstellung einer Zusammensetzung zur Behandlung von Haar/Kopfhaut, umfassend die Schritte:
i. Vormischen von aliphatischer Säure, wobei die aliphatische Säure C₈-C₂₄-Hydroxyl-substituierte aliphatische Säure umfasst, wobei die aliphatische Säure 12-Hydroxystearin umfasst, mit einem Fettacylisethionat-Produkt, welches Produkt 40 bis 80% eines oder mehrerer Fettacylisethionat-Salze, bezogen auf das Gewicht des Produkts, und 15 bis 50% einer oder mehrerer freier Fettsäuren und/oder der Salze der freien Fettsäure, bezogen auf das Gewicht des Produkts umfasst; und
ii. Kombinieren der Vormischung mit dem (den) verbleibenden Bestandteil(en) der Zusammensetzung zur Behandlung von Haar;
wobei sich die Fettsäure im Wesentlichen von der aliphatischen Säure unterscheidet.

## Revendications

1. Composition de traitement des cheveux/cuir chevelu comprenant :
a) un acide aliphatique comprenant un acide aliphatique substitué par un hydroxyle en C₈₋₂₄, où l'acide aliphatique comprend de l'acide 12-hydroxystéarique ;
b) un produit d'iséthionate d'acyle gras lequel produit comprend de 40 à 80 % d'un ou plusieurs sels d'iséthionate d'acyle gras en masse du produit et de 15 à 50 % d'un ou plusieurs acides gras libres et/ou des sels de l'acide gras libre en masse du produit ; et
c) au moins 30 % d'eau en masse de la composition ;
où l'acide gras est substantiellement différent de l'acide aliphatique.

2. Composition selon la revendication 1, où l'acide aliphatique est présent dans une quantité de 0,0001 à 5 % en masse de la composition, de préférence de 0,01 à 1 % en masse.

3. Composition selon l'une quelconque des revendications précédentes, où le produit d'iséthionate d'acyle gras est présent dans une quantité de 0,001 à 10 % en masse de la composition.

4. Composition selon l'une quelconque des revendications précédentes, où les sels d'iséthionate d'acyle gras présentent la formule de R²C(O)OR³SO₃M et les acides gras/sels présentent la formule de R⁴C(O)OX, où R² et R⁴ sont indépendamment choisis parmi des radicaux hydrocarbonés monovalents, linéaires ou ramifiés, saturés ou insaturés ayant de 5 à 31 atomes de carbone, R³ est indépendamment choisi parmi des radicaux hydrocarbonés divalents, linéaires ou ramifiés, saturés ayant de 2 à 6 atomes de carbone, M est un cation solubilisant, et X est l'hydrogène ou un cation solubilisant.

5. Composition selon la revendication 4, où R² et R⁴ sont indépendamment choisis parmi des radicaux hydrocarbonés monovalents, linéaires, saturés ou insaturés ayant de 5 à 24 atomes de carbone, et M est le sodium, et X est H ou le sodium.

6. Composition selon la revendication 4 ou la revendication 5, où au moins un de R² est identique à R⁴.

7. Composition selon l'une quelconque des revendications précédentes, où l'acide gras libre est un acide gras saturé sans substitution d'hétéroatome.

8. Composition selon l'une quelconque des revendications précédentes, où la composition comprend de plus un tensioactif anionique, de préférence le laurylsulfate de sodium et/ou lauryléthersulfate de sodium.

9. Composition selon l'une quelconque des revendications précédentes, où la composition comprend au moins un choisi parmi un agent de conditionnement des cheveux, un polymère de dépôt cationique, un actif anti-pelliculaire, un agent de mise en suspension, et une combinaison de ceux-ci.

10. Composition selon l'une quelconque des revendications précédentes où la composition est un shampoing.

11. Procédé de préparation d'une composition pour le traitement des cheveux/cuir chevelu comprenant les étapes de :
i. prémélange d'acide aliphatique où l'acide aliphatique comprend un acide aliphatique substitué par un hydroxyle en C₈₋₂₄, où l'acide aliphatique comprend de l'acide 12-hydroxystéarique, avec un produit d'iséthionate d'acyle gras lequel produit comprend de 40 à 80 % d'un ou plusieurs sels d'iséthionate d'acyle gras en masse du produit et de 15 à 50 % d'un ou plusieurs acides gras libres et/ou des sels de l'acide gras libre en masse du produit ; et
ii. combinaison du prémélange et du(des) constituant(s) restant(s) de la composition pour le traitement des cheveux ;
où l'acide gras est substantiellement différent de l'acide aliphatique.
